# EUROPÄISCHE PATENTSCHRIFT

(11) **EP 3 280 356 B1**
(45) Veröffentlichungstag und Bekanntmachung des Hinweises auf die Patenterteilung: **28.11.2018**
(21) Anmeldenummer: 16715497.0
(22) Anmeldetag: 06.04.2016
(51) Int. Cl.: A61F 2/20, A61M 16/04

(54) **SPRECHVENTIL MIT DECKELTEIL, UMFASSEND EIN VERSCHLUSSTEIL MIT EINEM ANSTEIGENDEN MITTELTEIL**
SPEAKING VALVE WITH COVER PART, COMPRISING A CLOSURE PART WITH A RISING CENTRAL PART
VALVE DE PHONATION COMPRENANT UNE PARTIE COUVERCLE POURVUE D'UNE PARTIE CLAPET À PARTIE MÉDIANE INCLINÉE VERS LE HAUT

(30) Priorität: 10.04.2015 DE 102015105496
(43) Veröffentlichungstag der Anmeldung: 14.02.2018
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2016/057489
(87) Internationale Veröffentlichungsnummer: WO 2016/162365

(56) Entgegenhaltungen:
- WO-A2-2008/132222
- DE-A1-102012 109 916

## Beschreibung

Die Erfindung betrifft ein Sprechventil für laryngektomierte oder tracheotomierte Menschen. Das Sprechventil weist ein Deckelteil, ein Gehäuseteil und einen Filter auf.
Sprechventile sind aus dem Stand der Technik bekannt. Diese werden zum Beispiel an eine Tracheostomakanüle oder ein Tracheostomapflaster angebracht, damit Menschen ohne Stimmbänder mittels einer Stimmprothese sprechen können. Durch Betätigen des Sprechventils wird Luft durch eine Stimmprothese, die in einer Fistel zwischen Trachea und Ösophagus angeordnet ist, geleitet. Auch wenn die Stimmbänder noch vorhanden sind ist es notwendig, dass die Luft nicht aus der Trachealkanüle entweicht, bevor diese die Stimmbänder erreicht.
Aus der US 4,582,058 ist bekannt, ein Sprechventil mittels eines Druckimpulses, der durch die Atmung des Patienten erfolgt, zu schließen und dadurch ein Sprechen zu ermöglichen. Jedoch haben sich diese Ventile als nicht besonders zuverlässig erwiesen.
Die WO 95/17138 A1 schlägt vor, das Sprechventil mit einer künstlichen Nase zu kombinieren. Diese künstliche Nase ist ein Filter, der Feuchtigkeit und Wärme der ausgeatmeten Luft auffängt und die in die Trachealkanüle einströmende Luft anfeuchtet und erwärmt.
Die EP 1 077 658 B1 beschreibt ein Stimmventil mit einem Filter, wobei das Stimmventil ein elastisches Gehäuse beziehungsweise elastisches Ventilelement aufweist und das Gehäuse beziehungsweise Ventilelement derart beispielsweise mittels Fingerdruck verformt werden kann, dass ein Gehäuseteil zur Auflage auf einem insbesondere durch eine Trachealkanüle oder ein Tracheostomapflaster gebildeten Ventilsitz kommt und das Ventil damit geschlossen wird.

Die WO2008/132222 offenbart ein weiteres Stimmventil mit Filter, welches durch Druck auf den Deckel geschlossen werden kann. Aufgabe der vorliegenden Erfindung ist es, ein Sprechventil zur Verfügung zu stellen, welches einfach aufgebaut und kostengünstig herzustellen ist

Die Aufgabe wird erfindungsgemäß gelöst mittels eines Sprechventils nach Anspruch 1. Weitere vorteilhafte Ausgestaltungen sind der nachfolgenden Beschreibung, den Figuren sowie den Unteransprüchen zu entnehmen. Die einzelnen Merkmale der beschriebenen Ausgestaltungen sind jedoch nicht auf diese beschränkt, sondern können unter einander und mit anderen Merkmalen zu weiteren Ausgestaltungen verknüpft werden.

Es wird ein Sprechventil für Laryngektomierte oder Tracheotomierte vorgeschlagen. Das Sprechventil umfasst ein Deckelteil, ein Gehäuseteil und einen Filter. Das Deckelteil ist mittels einer Rastverbindung an dem Gehäuseteil angeordnet. Das Deckelteil umfasst ferner ein Verschlussteil. Das Verschlussteil und das Deckelteil bilden ein (Bau-)Teil des Ventils. Das Verschlussteil ist vorteilhafterweise vollständig in einem durch das Gehäuseteil gebildeten Inneren angeordnet. Zumindest ein elastischer Bereich des Deckelteils fungiert als Rückstellelement.

Das Deckelteil umfasst des Weitern ein elastisches Material, welches insbesondere gummielastische Eigenschaften hat, wobei mittels Verformens, insbesondere ein Einbeulen nach proximal zumindest eines Bereiches des Deckelteils, das Deckelteil mit dem Verschlussteil das Sprechventil distal des Filters verschließt.

Vorteilhafterweise sind Deckelteil (mit Verschlussteil), Filter und/oder Gehäuseteil von einander trennbar, so dass insbesondere das Deckelteil oder der Filter austauschbar ausgestaltet ist. Deckelteil, Filter und Gehäuseteil sind somit gemäß einer vorteilhaften Ausführungsform separate Bauteile.

Die Rückstellkräfte, die bei einer Überführung des Sprechventils von einer zum Sprechen notwendigen Geschlossenstellung in eine Offenstellung benötigt werden, werden überwiegend, vorteilhafterweise im wesentlichen vollständig, bevorzugt vollständig, von dem aus einem elastischen Material gebildeten Deckelteil zur Verfügung gestellt. Dadurch entfallen vorteilhafterweise ansonsten notwendige Rückstellmittel wie Federn oder ähnliches. Da durch den distalen Verschluss der Filter allenfalls gering, insbsondere überwiegend nur in einem Teilbereich, der oberhalb eines durch das Gehäuseteil zur Verfügung gestellten Ventilsitz angeordnet ist, bevorzugt im Wesentlichen nur teilweise, weiter bevorzugt nicht komprimiert wird, bleibt dessen Funktion des Wärme- und Feuchtigkeitsaustausches so gut wie vollständig, wenn nicht vollständig erhalten.

Die in der vorliegenden Erfindung verwendeten Richtungsangaben sind gemäß der Erfindung in Bezug auf den bestimmungsgemäßen Einbau am Körper beziehungsweise an einer Trachealkanüle oder einem Tracheostomapflaster zu verstehen. Soweit in der vorliegenden Erfindung eine Mittelachse und eine Ausrichtung in Bezug auf diese Mittelachse des erfindungsgemäßen Sprechventils angesprochen ist, so ist von einer Anordnung der Mittelachse im Mittelpunkt des Sprechventiles auszugehen. Das Sprechventil ist dem Grunde nach radialsymmetrisch ausgebildet, insbesondere mit einem kreisrunden Gehäuseteil und weist daher in Aufsicht einen Mittelpunkt zumindest am Gehäuseteil auf, durch welchen die Mittelachse verläuft. Die Mittelachse verläuft parallel zu einer Innen- oder Außenkontur beziehungsweise einer Außenwandung des Gehäuseteils durch das erfindungsgemäße Sprechventil. Soweit im Bezug auf die Mittelachse der vorliegenden Erfindung von einer parallelen Anordnung eines Bauteiles oder Merkmales die Rede ist, bezieht sich dies eben auf die entsprechend dem Vorstehenden definierte Mittelachse und eine parallele Anordnung des Bauteils hierzu. Soweit in der vorliegenden Erfindung von einer vertikalen Ausrichtung, insbesondere in Bezug auf das Deckelteil, die Rede ist, insbesondere eines Außenrandes desselben, so ist hierunter ebenfalls eine im Wesentlichen parallele Anordnung mit Blick auf die Mittelachse zu verstehen. Soweit in der vorliegenden Erfindung von einer lateralen Anordnung, insbesondere in Hinblick auf das Deckelteil, insbesondere eine laterale Überragung einer Außenkontur des Gehäuseteils durch das Deckelteil, die Rede ist, so ist hierzu eine im Wesentlichen senkrechte Anordnung in Bezug auf die Mittelachse zu verstehen.

Unter dem Begriff "distal" wird im Sinne der vorliegenden Erfindung in Bezug auf ein Merkmal der erfindungsgemäßen Vorrichtung eine Anordnung beziehungsweise Verwendung desselben fern oder auch abgewandt oder gegenüberliegend eines Tracheostomapflasters oder einer Trachealkanüle, allgemeiner einer Hautoberfläche einer Person, die insbesondere solche Befestigungsmittel für die erfindungsgemäße Vorrichtung trägt, verstanden. Ein Verschluss distal des Filters, das heißt bei Überführung des Verschlussteils aus einer Offenstellung in eine Geschlossenstellung, bedeutet, dass der Filter vollständig unterhalb des Verschlussteils, das heißt proximal, angeordnet ist. Unter dem Begriff "proximal" wird im Sinne der vorliegenden Erfindung in Bezug auf ein Merkmal der erfindungsgemäßen Vorrichtung eine Anordnung oder Verwendung desselben nah oder auch zugewandt oder benachbart eines Tracheostomapflasters oder einer Trachealkanüle, allgemeiner einer Hautoberfläche einer Person, die insbesondere solche Befestigungsmittel für die erfindungsgemäße Vorrichtung trägt, verstanden.

"Einteilig" im Sinne der vorliegenden Erfindung heißt nicht, dass zum Beispiel Verschlussteil und Deckelteil materialeinheitlich ausgebildet sein müssen. Sie können beispielsweise aus unterschiedlichen Materialien hergestellt und miteinander gleich wie verbunden sein, zum Beispiel durch Klebung, durch mechanische Mittel etc. Auch in diesem Fall bilden beide zusammen ein einziges Bauteil des erfindungsgemäßen Sprechventils, was als einteilig im Sinne der vorliegenden Erfindung anzusprechen ist.

Das vorgeschlagene Sprechventil hat den Vorteil, dass sehr wenige Bauteile zur Realisierung des Sprechventils Verwendung finden. Das Sprechventil besteht im Wesentlichen nur aus drei Bauteilen, nämlich dem Filter, dem Gehäuseteil und dem Deckelteil, welches das Verschlussteil umfasst, und damit mit diesem ein Bauteil bildet. Darüber hinaus weist das erfindungsgemäße Sprechventil den großen Vorteil auf, dass aufgrund der spezifischen Ausgestaltung des Verschlussteiles Pfeifgeräusche insbesondere beim Einatmen vermieden werden, da durch den ansteigenden Mittelteil ein Raum zwischen dem Verschlussteil und dem Filter spezifisch vergrößert wird. Zudem treten durch die Ausbildung des Sprechventiles im Raum zwischen Verschlussteil und Filter Verwirbelungen auf, die eine gleichmäßige Durchfeuchtung und Erwärmung der Atemluft durch eine vollständige Ausnutzung der Kapazität des Filters ermöglichen. Aufgrund der spezifischen Ausgestaltung des Verschlussteils können insbesondere auch Einströmöffnungen, die im Gehäuseteil angeordnet sind, relativ klein gehalten werden. Durch diese Einströmöffnungen wird Atemluft eingeatmet oder wieder abgegeben. Die Einströmöffnungen können daher insbesondere eine Höhe parallel zu einer Mittelachse des erfindungsgemäßen Sprechventiles aufweisen, die recht gering ist, so dass sich letztendlich auch ein relativ flach bauendes erfindungsgemäßes Sprechventil vorteilhafterweise herstellen lässt.

Erfindungsgemäß weist das Verschlussteil auf der Stirnfläche des Außenrandes mindestens ein Lippenelement auf. Das Lippenelement ist bevorzugt auf der Stirnfläche als Verlängerung einer Außenwandung des Außenrandes des Verschlussteils ausgebildet, welche gegenüberliegend einer Innenwandung des Gehäuseteils angeordnet ist. Das Lippenelement ist bevorzugt elastisch ausgebildet. Es wirkt erfindungsgemäß mit dem Ventilsitz des Gehäuseteils zusammen. Vorteilhafterweise verbessert das Lippenelement eine abdichtende Wirkung des Verschlussteiles bei Überführung desselben durch die Ausübung eines Druckes auf den zumindest einen Bereich des Deckelteils, der elastisch ausgebildet ist, in die Schließstellung durch Zusammenwirken mit dem Ventilsitz, welcher bevorzugt durch das Gehäuseteil gebildet ist. Das Lippenelement ist vorzugsweise als geschlossener Ring ausgebildet, es können jedoch auch mehrere Lippenelemente, beispielsweise eins, zwei, drei, vier, fünf, sechs, sieben, acht, neun oder mehr auf der Stirnfläche des Außenrandes des Verschlussteiles angeordnet sein. Sobald mehrere Lippenelemente vorgesehen sind, sind diese bevorzugt durch kurze Bereiche unterbrochen, wobei das Lippenelement bei Überführung des Verschlussteils in eine Schließstellung des Sprechventiles Material in diese Bereiche verschiebt bei Zusammenwirkung mit dem Ventilsitz, so dass eine weitgehend abdichtende Wirkung erzielbar ist. Im Verhältnis zu einer Höhe des Außenrandes des Verschlussteiles beträgt die Höhe des Lippenelementes, ebenfalls parallel zu der Mittelachse des Sprechventiles bestimmt, 2 % bis etwa 20 % der Höhe des Außenrandes beziehungsweise dessen Außenwandung, bevorzugt etwa 3 % bis etwa 15 %, noch weiter bevorzug etwa 5 % bis etwa 12 %, der Höhe des Außenrandes des Verschlussteils.

In einer bevorzugten Ausführungsform weist der Außenrand des Ventilteils eine Außenwandung auf, die im Wesentlichen parallel zu einer Innenkontur beziehungsweise einer Innenwandung des Gehäuseteils angeordnet ist. Hierdurch kann eine sichere Führung des Verschlussteiles im Gehäuseteil bei Überführung des Sprechventils in Schließstellung und Rückstellung durch das elastische Material des Deckelteils in eine Offenstellung erzielt werden.

Weiter bevorzugt umfasst der Befestigungsbereich eine Ausnehmung, in welcher ein Befestigungsvorsprung des Deckelteils eingreifbar ist. Besonders bevorzugt ist die Ausnehmung des Befestigungsbereiches des Verschlussteiles als durchgehende Öffnung beziehungsweise Bohrung ausgebildet. Besonders bevorzugt ist der Befestigungsvorsprung des Deckelteils noppenartig ausgebildet, insbesondere als noppenartiger Vorsprung mit einer umlaufenden Nut. In dieser Nut ist vorteilhafterweise das Verschlussteil angeordnet, welches im Befestigungsbereich eine Höhe aufweist, die in etwa der Höhe der Nut entspricht. Besonders bevorzugt ist der Befestigungsvorsprung aus dem gleichen elastischen Material hergestellt wie der zumindest eine Bereich des Deckelteils, der durch Verformen eine Schließstellung des Sprechventils ermöglicht. Weiter bevorzugt weist der Befestigungsvorsprung einen Durchmesser auf, der groß genug ist, um einerseits abdichtend die Ausnehmung beziehungsweise Öffnung des Verschlussteiles zu verschließen, andererseits auch eine hinreichende Festigkeit durch das bevorzugt verwendete elastische Material zur Verfügung stellt, so dass ein sicherer Halt des Verschlussteils auf der Innenseite des Deckelteils sichergestellt ist.

Erfindungsgemäß ist vorgesehen, dass das Gehäuseteil distal einen Ventilsitz umfasst. Das Verschlussteil wirkt bei Betätigung des Sprechventils mit dem Ventilsitz, insbesondere über dessen untere Stirnfläche des Außenrandes, zusammen. Vorteilhafterweise wirken Sprechventil und Ventilsitz zusammen, so dass das Ventil schließt. Das eigentliche Ventil wird vom Verschlussteil und dem Ventilsitz gebildet. Der Ventilsitz ist vorzugsweise im Inneren des Gehäuseteils angeordnet, und ist bevorzugt als umlaufender Vorsprung unterhalb oder auf einer Höhe einer unteren Öffnungskante der Einströmöffnungen im Gehäuseteil angeordnet.

In einer besonders bevorzugten Ausführungsform wirkt die untere Stirnfläche des Verschlussteils zumindest teilweise mit dem Ventilsitz zusammen. Bevorzugt wirkt mindestens ein Flächenanteil von 50 % der Stirnfläche mit dem Ventilsitz im Gehäuseteil zusammen. Die untere Stirnfläche des Außenrandes des Ventilteils ist besonders bevorzugt parallel zu der Fläche des Ventilsitzes angeordnet. Erfindungsgemäß ist mindestens ein Lippenelement auf der unteren Stirnfläche des Außenrandes des Verschlussteiles angeordnet und wirkt ebenfalls mit dem Ventilsitz zusammen, und zwar bevorzugt vollständig, das heißt über die gesamte Stirnfläche des Lippenelements mit der Ventilsitzfläche, zur Verfügung gestellt durch das Gehäuseteil. Bei Ausübung eines stärkeren Druckes des Bedieners des Sprechventiles erfolgt dann ein Zusammenwirken auch der unteren Stirnfläche der Außenwandung des Verschlussteils im Übrigen mit dem Ventilsitz, das heißt der durch den Ventilsitz zur Verfügung gestellten Sitzfläche. Erfindungsgemäß wirkt das mindestens eine Lippenelement mit dem Ventilsitz zusammen.

Erfindungsgemäß ist vorgesehen, dass das Gehäuseteil distal des Ventilsitzes Einströmöffnungen umfasst. Dies hat den Vorteil, dass die einströmende Luft beim Einatmen den im Gehäuseteil angeordneten Filter vollständig durchströmt, bevor diese weiter durch die zumindest eine Ausströmöffnung in zum Beispiel eine Trachealkanüle beziehungsweise die Trachea hineinströmt. Der Ventilsitz ist vorteilhafterweise auf einer Ebene angeordnet wie das distale Ende des Filters, kann jedoch auch oberhalb oder unterhalb des distalen Endes des Filters angeordnet sein. Bei einer Anordnung unterhalb des distalen Endes des Filters erfolgt eine geringe Kompression im Wesentlichen des überstehenden Teils des Filters bei Überführung des Sprechventils aus einer Offenstellung in eine Geschlossenstellung. In einer weiteren Ausgestaltung ist vorgesehen, dass der Ventilsitz nach distal von der Oberfläche des Filters beabstandet ist. Eine weitere Variante sieht vor, dass der Ventilsitz derart im Gehäuse angeordnet ist, dass dieser proximal von dem distalen Ende des Filters beabstandet ist, so dass bei einem Verschließen des Sprechventils der Kolben den Filter teilkomprimiert. Auf die insbesondere teilweise Verdichtung des Filters während des Verschließvorgangs wird weiter unten genauer eingegangen.

In einer besonders bevorzugten Ausführungsform weist die mindestens eine Einströmöffnung in Richtung der Mittelachse des erfindungsgemäßen Sprechventils eine Höhe auf, die etwa gleich oder kleiner einer Höhe des Außenrandes beziehungsweise der Außenwandung, gegebenenfalls der Außenwandung einschließlich des mindestens einen Lippenelementes, des Verschlussteils ist. Hierdurch wird sichergestellt, dass auch im Bereich der Einströmöffnungen das Verschlussteil sicher innerhalb des Gehäuseteils geführt wird, soweit mindestens ein Bereich des Deckelteils verformt wird bei Bedienung des erfindungsgemäßen Sprechventiles durch eine Person.

Erfindungsgemäß ist vorgesehen, dass das Gehäuseteil Einströmöffnungen umfasst, die bei Betätigung des Sprechventils mittels des Verschlussteils verschließbar sind. Insbesondere ist das Verschlussteil so im Gehäuse geführt, dass dieser bei Betätigung des Sprechventils den Luftdurchtritt der Einströmöffnungen von innerhalb des Gehäuses zumindest teilweise versperrt. Das Sprechventil wird somit bei dieser Ausführungsform durch alternative oder gemeinsam wirkende Verschlussmechanismen verschlossen: Zum einen versperrt das Verschlussteil zumindest teilweise den Luftdurchtritt durch die Einströmöffnung. Weiterhin ist das Verschlussteil dergestalt ausgestattet, dass dieses mit dem Ventilsitz oder dem Filter zusammenwirkt, wenn dieses in das Gehäuseteil hineingedrückt wird, so dass eine Sperrung des Luftstroms innerhalb des Gehäuses vorgenommen wird. Eine weitere Verschlusstechnik ist, dass das Verschlussteil auf dem Filter aufliegt, insbesondere im Bereich der unteren Stirnfläche des Außenrandes des Verschlussteils, und bevorzugt einen Außenumfang des Filters im eingesetzten Zustand überragt, so dass ein Luftdurchtritt durch den Filter weitgehend verhindert wird. Dies hat den Vorteil, dass eine Abdichtung des Sprechventils sicher erfolgt. Im Bereich des Verschlussteils, insbesondere zentral, können im Deckelteil und gegebenenfalls auch dem Verschlussteil alternativ oder zusätzlich eine oder mehrere Öffnungen vorgesehen sein, damit Luft durch das Ventil strömen kann. Diese werden bei einer Schließung des Ventils durch insbesondere einen Finger des Benutzers bevorzugt verschlossen.

Gemäß einer weiteren Ausführungsform ist vorgesehen, dass das Deckelteil auf dem Gehäuseteil lösbar angeordnet ist. Beispielsweise können für ein Gehäuseteil unterschiedliche Deckelteile vorgesehen sein, die verschiedene Eigenschaften beispielsweise im Material, in der Festigkeit und/oder in der Formgebung aufweisen. Auch können für Schmuckzwecke unterschiedliche Farben und Verzierungen vorgesehen sein, die das Deckelteil aufweist, so dass eine variable Gestaltungsmöglichkeit des Sprechventils und somit des Tracheostomas gegeben ist.

Weiterhin vorteilhaft an der Austauschbarkeit des Deckelteils ist, dass bei einer Materialermüdung des Deckelteils durch die Benutzung und somit ständige Verformung das Deckelteil ausgetauscht werden kann, wobei Gehäuse und Filter verbleiben können. Weiterhin ist vorteilhaft, dass sofern das Deckelteil lösbar am Gehäuse angeordnet ist, der Filter leicht aus dem Gehäuse entnommen und ausgetauscht werden kann.

Erfindungsgemäß ist vorgesehen, dass das Deckelteil mittels einer Rastverbindung auf dem Gehäuseteil angeordnet ist. Aber auch andere Möglichkeiten einer lösbaren Verbindung, beispielsweise zur Verfügung gestellt durch eine oder mehrere Klettverbindungen oder Klebeverbindungen, sind möglich. Die Rastverbindung kann derart ausgeführt sein, dass das Deckelteil mindestens ein, zwei, drei, vier oder mehr Rastelemente, beispielsweise in Form von Rastnasen, aufweist, die an einer Innenfläche des Deckelteiles, der Seitenwandung des Gehäuseteiles zugeordnet, angeordnet sind. Dabei kann vorgesehen sein, dass die Rastelemente einen oberen Rand des Gehäuseteiles umfassen in einem Bereich desselben, in dem Einströmöffnungen vorgesehen sind. An diesem Rand können entsprechende Aufnahmen, Vorsprünge oder Ausnehmungen oder aber auch Durchbrüche vorgesehen sein, in welche das mindestens eine Rastelement des Deckelteiles einrastet oder dieses umgreift. Umgekehrt können entsprechende Rastelemente auch im distalen Endbereich des Gehäuseteiles angeordnet sein, beispielsweise Rastnasen, die in entsprechende Ausnehmungen oder Vorsprünge, angeordnet an der Unterseite des Deckelteiles, einrasten oder diese umgreifen.

In einer besonders bevorzugten Ausführungsform weist das Deckelteil auf seiner Innenseite angeordnet mindestens ein Rastelement auf, welches mindestens eine zumindest teilweise umlaufende Nut bildet. Besonders bevorzugt ist dabei das mindestens eine Rastelement im Querschnitt des Sprechventils gesehen in einer Ebene, die durch die Mittelachse derselben verläuft, L-förmig ausgebildet. Die Nut wird besonders bevorzugt durch einen kurzen Schenkel des L gebildet, und andererseits durch eine durch die Innenseite des Deckelteils zur Verfügung gestellte Fläche. Besonders bevorzugt ist die Nut im Querschnitt gesehen ausgebildet in Form eines liegenden U oder aber auch halbkreisförmig oder ähnlich. Auf jeden Fall wird eine obere Begrenzung der Nut gebildet durch eine Teilfläche der Innenseite des Deckelteils. Eine untere Begrenzung der Nut wird gebildet durch das Rastelement. Besonders bevorzugt ist genau ein Rastelement ringförmig umlaufend auf der Innenseite des Deckelteiles angeordnet. Weiter bevorzugt bildet das Rastelement genau eine umlaufende kreisförmige Nut zusammen mit einer Teilfläche der Innenseite des Deckelteils aus. Es kann jedoch auch vorgesehen sein, dass in der Nut einige Unterbrechungselemente angeordnet sind, beispielsweise zwei, drei, vier oder mehr, die auch als Füllstücke der Nut angesprochen werden können. In diesem Fall ist das Deckelteil derart ausgebildet, dass es passgenau auf ein entsprechend ausgebildetes Gehäuseteil aufsetzbar, das heißt verrastbar, ist. Dementsprechend ist bevorzugt in einem oberen Rand des Gehäusteils entsprechende Rastvorsprünge mit Ausnehmung vorgenommen, wobei die Breite der Ausnehmung in etwa den Füllelementen in der durch das Rastelement auf der Innenseite des Deckelteils gebildeten Nut entsprechen.

Die lösbare Verbindung zwischen Deckelteil und Gehäuseteil ist vorzugsweise derart ausgestaltet, dass im Verbindungsbereich keine Luft zwischen Deckelteil und Gehäuseteil hindurch ein- oder ausströmen kann. In einer bevorzugten Ausführungsform ist vorgesehen, dass an der Innenseite des Deckelteils drei oder vier Rastnasen oder ein Rastelement, das eine umlaufende Nut ausbildet, angeordnet sind beziehungsweise ist. Die drei oder vier Rastnasen sind auf einen zur Erzielung einer hinreichend festen Verbindung auf der Innenseite des Deckelteiles angeordneten Bund oder Wulst angeordnet, der in seiner Dimensionierung, das heißt insbesondere in seinem Durchmesser, dem Durchmesser des Gehäuseteils entspricht, und den oberen Rand der Seitenwandung des Gehäuseteils in etwa ab- oder überdeckt. Die Rastelemente beziehungsweise -nasen greifen bevorzugt in entsprechende Ausnehmungen am oberen Rand des Deckelteils ein oder umgreifen dort vorgesehene Vorsprünge. Der Wulst oder Bund oder das Rastelement, ausbildend mindestens eine zumindest teilweise umlaufende Nut, wie oben dargelegt, kann nur gering beabstandet von einem Außenrand des Deckelteils angeordnet sein. Das Deckelteil kann aber beispielsweise mit dem Gehäuseteil vernietet oder verschraubt sein, oder aber auch beispielsweise in Form einer Bajonettverschlusses mit dem Gehäuseteil verbunden sein. Eine weitere Variante sieht vor, dass das Deckelteil auf das Gehäuseteil aufgeschraubt wird. Weiterhin sieht eine Ausgestaltung vor, dass das Deckelteil mittels eines Bajonettverschlusses auf dem Gehäuseteil aufgebracht wird. Es kann eine weitere Variante vorgesehen sein, bei der das Deckelteil mit dem Gehäuseteil verklebt oder verschweißt wird.

Die unterschiedlichen Einstellmöglichkeiten beispielsweise in der Elastizität des Materials insbesondere des gummielastischen Materials, oder in der Formgebung können die Eigenschaften des Sprechventils beeinflussen. So kann beispielsweise die Rückstellkraft durch die Materialeigenschaften und die Dicke des Materials des Deckelteils als auch durch die Formgebung beeinflusst werden. In einer Ausgestaltung ist vorgesehen, dass unterschiedlich eingestellte Deckelteile zur Verfügung gestellt werden, die austauschbar an dem Gehäuseteil angeordnet werden und für verschiedene Verwendungszwecke einsetzbar sind.

Erfindungsgemäß ist das Deckelteil zumindest teilweise aus einem elastischen Material gebildet. Vorteilhafterweise ist das elastische Material des Deckelteils ein gummielastisches Material. Bevorzugt ist das elastische Material des Deckelteiles ein linear-elastisches Material. Als gummielastisches Material kann insbesondere Naturkautschuk oder aber Synthesekautschuk, insbesondere Butyl-Kautschuk, oder aber Ethylen-Propylen-Dien-Kautschuk (EPDM), eingesetzt werden. Aber auch der Einsatz von Silikonmaterialien, insbesondere medizinischen Silikonmaterialien, ist möglich. Grundsätzlich kann das gummielastische Material des Deckelteiles ein Elastomer sein, beispielsweise ein thermoelastisches Elastomer auf Olefin- oder Urethanbasis, besonders bevorzugt ein zumindest teilweise vernetztes thermoplastisches Elastomer auf Olefinbasis, ein Polyesterelastomer, ein thermoplastischer Copolyester, ein Styrolblock-Polymere oder ein thermoplastisches Copolymere. Anstatt aus einem gummielastischen Material hergestellt zu sein, welches aus einem Polymer gebildet ist, kann das Deckelteil zumindest teilweise aus einem linear-elastischem Material, insbesondere einem Metall, gebildet sein, welches eine hinreichende Elastizität aufweist. Aber auch andere Materialien, die eine hinreichende Elastizität zur Verfügung stellen, sind möglich. Besonders bevorzugt besteht das Deckelteil aus einem elastischen, bevorzugt gummielastischem Material, weist mithin kein weiteres Material auf. Das Deckelteil kann dabei eine gleichmäßige Stärke aufweisen, die Stärke kann jedoch insbesondere im Bereich des Überganges von einer horizontalen in eine vertikale Erstreckung des Deckelteiles verringert sein, und/oder ebenso in einem Bereich, durch welchen durch die Benutzer mittels eines Fingers eine Überführung des Sprechventiles aus der Offenstellung in die Geschlossenstellung und zurück erfolgt.

Erfindungsgemäß ist vorgesehen, dass zumindest ein elastischer Bereich des Deckelteils als Rückstellelement fungiert. Gemäß einer Ausgestaltung ist das elastische Material des Deckelteils ein gummielastisches Material. Wird dieses eingedrückt, um das Verschlussteil nach proximal innerhalb des Gehäuses zu verschieben und das Sprechventil somit zu schließen, bewirkt die dem gummielastische Material inhärente Rückstellkraft, dass das Sprechventil, sobald die Kraft auf das Deckelteil verringert wird, wieder öffnet. Eine weitere Ausgestaltung sieht vor, dass das elastische Material des Deckelteils ein linear elastisches Material, beispielsweise ein Metall, ist. Das Deckelteil kann aus einem oder mehreren Materialien bestehen. Insbesondere ist in einer Ausgestaltung vorgesehen, dass Materialien mit unterschiedlichen elastischen Eigenschaften vorgesehen sind und beispielsweise das Verschlussteil ein anderes Material, insbesondere ein rigides, das heißt nicht elastisches Material, oder ein weniger elastisches Material aufweist als das Deckelteil. Besonders bevorzugt ist dabei beispielsweise dass Verschlussteil ausgebildet aus einem Silikonmaterial, insbesondere einem medizinischen Silikon, was eine größere Festigkeit, das heißt Rigidität, aufweist, als das Material des Deckelteils. Das Deckelteil selbst ist vorzugsweise aus einem elastischen Material gebildet wie oben beschrieben. In einer weiteren vorteilhaften Ausgestaltung ist vorgesehen, dass das Deckelteil, und somit auch das Verschlussteil, aus einem elastischen, weiter bevorzugt identischen elastischen, Material bestehen. Bevorzugt kann das Deckelteil mit dem Verschlussteil in einem 1-Komponenten-Spritzverfahren hergestellt werden. Bei Verwendung eines identischen, elastischen Materials kann zum Beispiel über die Wandstärke oder sonstige mechanische Mitte! wie zum Beispiel Sicken oder Versteifungsstreben, Hohlräume o.ä. die Elastizität des Deckelteils und des Verschlussteils beeinflusst werden, so dass diese trotz gleichem Material unterschiedliche Elastizitätsmodule aufweisen.

Weiterhin sieht eine Ausgestaltung vor, dass das Verschlussteil zumindest teilweise aus einem rigideren Material gebildet ist als das Deckelteil, das heißt ein biegesteiferes oder weniger oder nicht elastisches Material, insbesondere im Bereich der Fläche, die mit dem Ventilsitz zusammenwirkt, insbesondere der unteren Stirnfläche des Außenrandes des Verschlussteils. Die Verwendung eines rigiden oder rigideren Materials für das Verschlussteil hat den Vorteil, dass dieses bei der Führung durch das Gehäuseteil während der Betätigung sich nicht oder nur unwesentlich verformt. Somit kann, auch wenn der Benutzer das Deckelteil nicht mittig drückt, ein vollständiger Verschluss des Sprechventils erfolgen, da das Verschlussteil nicht einseitig beziehungsweise ungleichmäßig verformt in das Gehäuseteil eindringt und einen nur unzureichenden Verschluss bewirkt. In einer weiteren Ausgestaltung ist vorgesehen, dass die Rigidität des Verschlussteils insbesondere mittels Verwendung von elastischem oder gummielastischen Vollmaterial erfolgt beziehungsweise durch dieses eingestellt ist. Das Verschlussteil kann einstückig mit dem Deckelteil, insbesondere auch materialeinheitlich, ausgebildet sein. Bevorzugt ist das Verschlussteil aus einem Material mit einem geringeren Elastizitätsmodul, also einem rigideren, bevorzugt rigiderem, Material, gefertigt als das Deckelteil. Besonders bevorzugt ist das Verschlussteil auf seiner dem Deckelteil zugewandten Fläche mit mindestens einer Ausnehmung, die auch als Öffnung oder Bohrung ausgebildet sein kann, oder mindestens einem Vorsprung versehen. Umgekehrt ist bevorzugt auf der Unterseite des Deckelteils, welche dem Verschlussteil zugewandt ist, mindestens ein Vorsprung oder mindestens eine Ausnehmung, die bevorzugt nicht als Öffnung oder Bohrung ausgebildet ist, vorgesehen. Ausnehmung und Vorsprung von Deckelteil und Verschlussteil wirken, beispielsweise durch Form- oder Kraftschluß, insbesondere durch Klebung oder mechanische Verrastung oder ähnliches, zusammen, so dass ein einstückiges Bauteil aus Deckelteil und Verschlussteil, die einteilig verbunden sind im Sinne der vorliegenden Anmeldung, erhalten ist.

Das Deckelteil ist vorteilhafterweise hut- oder kappenmäßig ausgebildet. Hierunter ist eine Ausgestaltung des Deckelteiles derart zu verstehen, dass das Deckelteil die Seitenwandung des Gehäuseteiles zumindest teilweise, bevorzugt vollständig (das heißt nach allen Seiten) überragt. Das Überragen der Seitenwandung des Gehäuseteils erfolgt dabei vorteilhafterweise in einer Richtung im Wesentlichen parallel zu einer Hautoberfläche des Körpers beziehungsweise im Wesentlichen senkrecht zu der Mittelachse des erfindungsgemäßen Sprechventils. Weiter bevorzugt erfolgt die Überragung der Seitenwandung des Gehäuseteils vertikal, das heißt in Richtung der vertikalen Erstreckung des Gehäuseteiles von seinem proximalen Ende zu seinem distalen Ende, beziehungsweise im Wesentlichen parallel zu der Mittelachse des erfindungsgemäßen Sprechventils. Das vertikale Überragen erfolgt bevorzugt über eine Teillänge der vertikalen Erstreckung des Gehäuseteils. Das Deckelteil kann in dem die Seitenwandung des Gehäuseteils überragenden Bereich auch Öffnungen, insbesondere schlitzförmig ausgebildete, aufweisen. Öffnungen können aber auch im Verformungsbereich des Deckelteils angeordnet sein, insbesondere genau eine Öffnung, die bei Überführung in die Geschlossenstellung durch den Nutzer verschlossen wird. In einer Ausgestaltung ist vorgesehen, wenn das Verschlussteil, was möglich ist, nicht mit seiner Oberseite, die der Unterseite des Deckelteils zugewandt ist, vollständig oder teilweise mit der Unterseite des Deckelteils verbunden ist, sondern insbesondere etwas beabstandet, beispielsweise durch eine Art Befestigungskreuz, in dessen Kreuzungspunkt mindestens eine Ausnehmung oder mindestens ein Vorsprung zur Befestigung an der Unterseite des Deckelteils vorgesehen ist. Das Deckelteil kann auf seiner Unterseite mindestens einen Vorsprung oder mindestens eine Ausnehmung aufweisen, die mit den entsprechenden Mitteln des Verschlussteils zusammenwirken. Beispielsweise kann der Vorsprung stift- oder stabförmig ausgebildet sein und die Ausnehmung als Sackloch, das bevorzugt dem Vorsprung angepasst ist. Bevorzugt erfolgt eine form- und/oder kraftschlüssige Verbindung zwischen Deckelteil und Verschlussteil.

Der Befestigungsbereich des Verschlussteils umfasst neben einer Ausnehmung, die bevorzugt als Öffnung beziehungsweise Bohrung ausgebildet ist, eine diese umfassende Verstärkung, die beispielsweise wulstartig ausgebildet sein kann. Die Verstärkung ist als Materialverdickung beziehungsweise -verstärkung anzusprechen. Insbesondere ist eine innenwandung der Ausnehmung beziehungsweise Öffnung derart ausgebildet, dass diese in eine an einem Befestigungsvorsprung des Deckelteils angeordnete umlaufende Nut form- und/oder kraftschlüssig eingreift.

Vorteilhafterweise weist das Deckelteil von proximal nach distal, und damit parallel zu der Mittelachse des erfindungsgemäßen Sprechventils, im unmontierten Zustand, das heißt gelöst vom Gehäuseteil, eine Höhe auf, die mindestens 10 % bis etwa 80 %, bevorzugt etwa 15 % bis etwa 65 %, weiter bevorzugt etwa 25 % bis etwa 30 %, noch weiter bevorzugt etwa 30 % bis etwa 48 % einer Höhe des Gehäuseteils entspricht. Im montierten Zustand, das heißt, wenn das Deckelteil auf dem Gehäuseteil angeordnet ist, bevorzugt verrastet ist, überragt ein Teil des Deckelteils vertikal das Gehäuseteil, besonders bevorzugt wird durch das Deckelteil eine vertikale Abdeckung der mindestens einen Einströmöffnung im Gehäuseteil zumindest zu 50 %, bevorzugt mindestens 80 %, weiter bevorzugt mindestens 90 % und noch weiter bevorzugt vollständig erzielt. Die vorstehenden Prozentangaben verstehen sich dabei bei Betrachtung des erfindungsgemäßen Sprechventiles mit auf dem Gehäuseteil angeordneten Deckelteil in einer Richtung entlang der Mittelachse, und zwar parallel zu dieser. Betrachtet man das Sprechventil von der Seite, so wird durch das Deckelteil bevorzugt die mindestens eine Einströmöffnung nahezu vollständig abgedeckt, das heißt es ist allenfalls noch ein Spalt einer Einströmöffnung für einen Betrachter bei seitlicher Ansicht des erfindungsgemäßen Sprechventils sichtbar.

Die Höhe des Deckelteils von proximal nach distal wird bestimmt einerseits durch eine untere Stirnfläche des Randes, der auch als zervikaler Rand angesprochen werden kann, andererseits durch die distale Oberfläche desselben, die am weitesten vom Tracheostoma absteht.

Soweit im Rahmen der vorliegenden Erfindung der Begriff "etwa" verwendet wird, so ist darunter ein Toleranzbereich zu verstehen, den der angesprochene Fachmann auf dem vorliegenden Gebiet für üblich erachtet, insbesondere ein Toleranzbereich von +/- 20 %, bevorzugt +/- 10 %, weiter bevorzugt +/- 5 %, jeweils bezogen auf den in Rede stehenden Wert oder Wertebereich.

In einer bevorzugten Ausführungsform überragt das Deckelteil das Gehäuseteil sowohl lateral als auch vertikal. Weiter bevorzugt umfasst das Deckelteil einen zervikalen Rand. Der zervikale Rand, damit die untere Stirnfläche des Randes des Deckelteils, der dem Hals eines Trägers des erfindungsgemäßen Sprechventiles zugewandt ist, ist vorzugsweise abgerundet ausgebildet, so dass keine Hautirritation auftreten, wenn bei Bewegung des Benutzers ein Hautkontakt erfolgte.

Insbesondere weist das Deckelteil bei lateraler Draufsicht eine runde, eine ovale oder andersartig gerundete oder eine rechteckige Formgebung auf, wobei bei einer eckigen Formgebung die Ecken vorteilhafterweise abgerundet sind. Vorteilhafterweise sind alle Kanten des Deckelteiles abgerundet, um insbesondere Verletzungen oder Hautirritierungen zu vermeiden. Die untere Stirnfläche des Randes des Deckelteils, welches horizontal und vertikal das Gehäuseteil, insbesondere eine Außenkontur desselben, überragt, liegt vorteilhafterweise in etwa auf der Höhe einer unteren Öffnungskante der mindestens einen Einströmöffnung.

Vorteil eines jeden der Merkmale, die vorstehend im Zusammenhang mit der Ausgestaltung des Deckelteiles beschrieben sind, ist eine Führung der Luft beim Einatmen am Hals entlang, so dass die Luft, bevor sie das Innere des Sprechventiles und insbesondere den Filter erreicht, vorgewärmt ist. Je nach Ausgestaltung des Deckelteiles, insbesondere im Hinblick auf die Anordnung des zervikalen Randes eher nah an der Seitenwandung des Gehäuseteiles oder eher entfernt von dieser, als auch durch die Anordnung des Deckelteiles mit dem zervikalen Rand derart, dass der zervikale Rand nahe oder weiter beabstandet dem Hals des Benutzers zugeordnet ist, kann sowohl das Volumen des aufzunehmenden Luftstromes als auch die Vorwärmung desselben beeinflusst werden.

Besonders vorteilhaft ist, wenn das Deckelteil distal, und dort insbesondere in einem zentralen Bereich, keine Öffnung aufweist, die einen Luftdurchtritt von distal nach proximal durch den Filter hindurch ermöglicht. Das Gehäuseteil umfasst vorzugsweise eine oder mehrere Einströmöffnungen in einer Seitenwand desselben. Hierdurch ist sichergestellt, dass die Luft im Wesentlichen am Hals entlanggeführt wird, bevor sie in das Gehäuseteil eintritt. Alternativ oder zusätzlich kann jedoch auch vorgesehen sein, dass das Deckelteil mindestens eine distale Öffnung, insbesondere in einem Bereich, der dem Inneren des Gehäuseteils zugewandt ist, aufweist. Durch die mindestens eine distale Öffnung kann zumindest ein Teil der eingeatmeten Luft direkt in das Gehäuse beziehungsweise durch den Filter dringen, so dass die Luft erfeuchtet und erwärmt in die Trachea beziehungsweise in die Trachealkanüle eindringen kann

In einer weiteren Variante des Sprechventils ist vorgesehen, dass der Filter mit Übermaßpassung oder über Übergangspassung in dem Gehäuseteil angeordnet ist. Vorteilhafterweise hat der Filter gemäß einer Ausgestaltung kein Spiel im Gehäuse. Dies verhindert einen Bypass von ein- und ausgeatmeter Luft am Filter vorbei. Weiterhin hat der derart angepasste Filter den Vorteil, dass dieser durch den wechselnden Luftdruck nicht im Gehäuse verschoben wird. Aber auch eine Anordnung des Filters im Gehäuse unter Bildung eines Spaltes zwischen der Außenwandung und der Innenwandung des Gehäuseteiles ist möglich. Der Filter kann durch ein Gehäuseteil gehalten sein durch Einklebung oder ähnliches, aber auch beispielsweise nur durch die Quetschpassung. Es können aber auch an der Innenwandung des Gehäuseteils Haltemittel angeordnet sein, die ein Eindringen in das Filtermaterial ermöglichen. Diese können beispielsweise als kegelstumpfförmige Vorsprünge ausgestaltet sein. Aber auch jede andere Ausgestaltung der Haltemittel ist möglich, soweit diese nur einen Halt des Filters im Gehäuseteil sicherstellen.

Ein großer Vorteil des vorgeschlagenen Sprechventils ist, dass der Filter nicht oder nur teilweise, insbesondere aufgrund eines den Ventilsitz möglicherweise überragenden Teils des Filters, beim Schließen des Sprechventils komprimiert wird. Die Komprimierung des Filters hat in den aus dem Stand der Technik bekannten künstlichen Nasen beziehungsweise Sprechventilen den Nachteil, dass der Filter ausgedrückt und somit seine Feuchtigkeit verliert. Weiterhin besteht grundsätzlich die Gefahr, dass das Filtermaterial in die Trachealkanüle gedrückt und im schlimmsten Fall von dem Benutzer respiriert wird.

In einer Ausführungsform ist vorgesehen, dass der Filter durch die Betätigung des Sprechventils teilkomprimiert wird. Bei einem Verschließen kann es in einer Ausgestaltung versehentlich oder absichtlich oder durch die Toleranzen des Sprechventils zu einer Teilkomprimierung des Filters kommen. Das Sprechventil ist derart ausgestaltet, dass eine Komprimierung von etwa 0 oder 1 Vol.-% bis etwa 50 Vol.-%, insbesondere etwa 0 oder 1 Vol.-% bis etwa 35 Vol.-%, bevorzugt etwa 1 Vol.-% bis etwa 50 Vol.-%, weiterhin bevorzugt etwa 1 Vol.-% bis etwa 35 Vol.-%, besonders bevorzugt 1 Vol.-% bis etwa 25 Vol.-% vorgesehen ist. Die Komprimierung kann beispielsweise durch das Deckelmaterial oder durch die Geometrie des Deckels eingestellt werden. Hierdurch wird dem Benutzer durch die üblicherweise aufgewendete Kraft, beispielsweise etwa 5 N bis etwa 15 N vorgegeben, wann ein Weitereindrücken nicht mehr erforderlich ist. Versucht der Benutzer ein weiteres Eindrücken des Deckelteils, kommt es gewöhnlicher Weise zu einem Unwohlsein durch den Druck auf das Tracheostoma beziehungsweise das umliegende Gewebe oder die Trachealkanüle. Das Sprechventil ist so ausgestaltet, dass dieses schon schließt, bevor die üblicherweise aufgewendete Kraft von beispielsweise etwa 5 N bis etwa 15 N erreicht ist. Die Einstellung der Komprimierung des Filters wird vorteilhafterweise durch die Anordnung des Ventilsitzes im Gehäuseteil, insbesondere in einer bestimmten Höhe innerhalb des Gehäuseteils, eingestellt. In einer weiteren Ausgestaltung ist vorgesehen, dass mittels eines am oder im Gehäuseteil angeordneten Gegenlagers eine Kompression des Filters begrenzt ist. Vorzugsweise kann das Verschlussteil nur bis zum Gegenlager in das Sprechteil hineingedrückt werden. Die Vorsehung eines Gegenlagers verhindert ein Durchdrücken des Filters in die Trachealkanüle und somit eines Respirieren des Filters. Weiterhin wird durch die nur begrenzte Kompression ein Ausdrücken der Feuchtigkeit des Filters vermindert bis verhindert. Das Gegenlager kann beispielsweise als Stab oder Wand, der oder die sich von proximal nach distal durch den Filter erstreckt und am Gehäuse in irgendeiner Form angeordnet ist, realisiert sein. Eine weitere mögliche Ausgestaltung sieht vor, dass das Gegenlager als ein zumindest teilweise umlaufender Rand ausgestaltet ist, der vorzugsweise ungleich des Ventilsitzes ist.

Des Weiteren betrifft die vorliegende Erfindung ein Verschlussteil, wie vorstehend beschrieben im Zusammenhang mit dem erfindungsgemäßen Sprechventil.

Weitere vorteilhafte Ausgestaltungen gehen aus den nachfolgenden Zeichnungen hervor. Die dort dargestellten Abbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die der Figurenbeschreibung angegebenen Bezugszeichen den Schutzbereich der vorliegenden Erfindung nicht beschränken, sondern lediglich auf die in den Figuren bezeichneten Ausführungsbeispiele verweisen. Es zeigen:
- Fig. 1: ein Sprechventil in einer ersten Ausführungsform in einer Schnittansicht im unbetätigten Zustand;
- Fig. 2: ein Sprechventil in einer zweiten Ausführungsform in einer Schnittansicht im unbetätigten Zustand, angeordnet auf einen Tracheostomapflaster;
- Fig. 3: das Sprechventil gemäß Fig. 2 in einer perspektivischen Ansicht von schräg oben;
- Fig. 4: das Sprechventil gemäß Fig. 2 in perspektivischen Ansicht von schräg unten;
- Fig. 5: ein Gehäuseteil zum Einsatz an einem erfindungsgemäßen Sprechventil in einer perspektivischen Ansicht;
- Fig. 6: das Gehäuseteil gemäß der Fig. 5 in einer Draufsicht;
- Fig. 7: das Gehäuseteil gemäß Fig. 5 in einer perspektivischen Ansicht von schräg unten;
- Fig. 8: ein Deckelteil zur Verwendung in einem erfindungsgemäßen Sprechventil in einer perspektivischen Ansicht, nach oben gedreht im Vergleich zu einer montierten Position des Deckelteils auf einem Gehäuseteil;
- Fig. 9: eine Draufsicht auf das Deckelteil gemäß Fig. 8;
- Fig. 10: ein erfindungsgemäßes Verschlussteil 3 zur Verwendung in einem erfindungsgemäßen Sprechventil, insbesondere einem solchen gemäß den Fig. 1 bis 4, in einer perspektivischen Ansicht; und
- Fig. 11: eine Draufsicht auf das Verschlussteil gemäß Fig. 10;

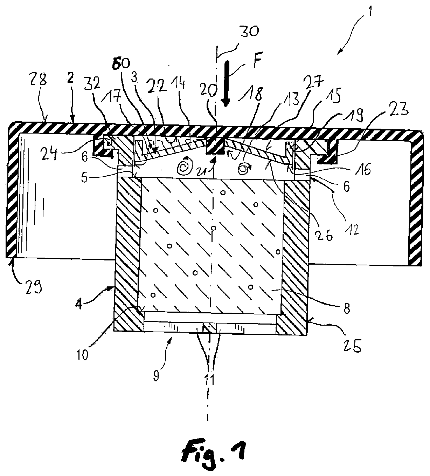

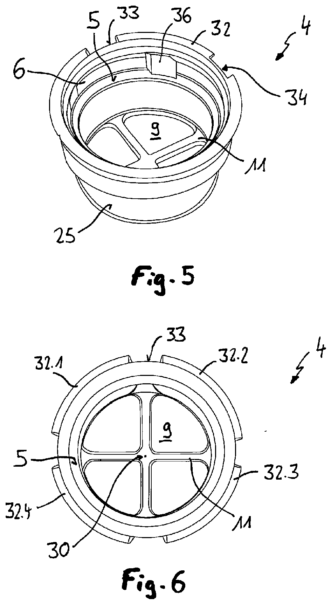

Fig. 1 zeigt ein Sprechventil 1 für laryngektomierte oder tracheotomierte Menschen in einer Schnittansicht in einer ersten Ausführungsform mit einem Deckelteil 2, einem Gehäuseteil 4 und einem Filter 8. Das Deckelteil 2 umfasst ein kreis- oder scheibenförmiges Verschlussteil 3, das mit dem Deckelteil 2 verbunden ist. In der hier gezeigten Ausgestaltung ist das Deckelteil 2 vollständig aus einem elastischen Material gefertigt. Das Deckelteil 2 ist am distalen Ende des Gehäuseteils 4 angeordnet. Vorzugsweise ist eine Rastverbindung vorgesehen, um das Deckelteil 2 an dem Gehäuseteil 4 zu befestigen. Hierzu sind an einer Innenseite 22 des Deckelteils 2 Rastelemente 23 ausgebildet, welche im Querschnitt gesehen in etwa L-förmig ausgebildet sind. Bevorzugt ist genau ein ringförmig auf der Innenseite 22 des Deckelteils 2 umlaufend ausgebildetes Rastelement 23 vorgesehen. Dieses bildet zusammen mit einer Innenfläche der Innenseite 22 des Deckelteils 2 eine im Querschnitt gesehen in Form eines liegenden U ausgebildete umlaufende Ringnut 24 aus. In diese umlaufende Ringnut 24 kann ein an einem oberen Rand 34 des Gehäuseteils 4 (siehe beispielsweise Fig. 5) angeordneter Rastvorsprung 32 des Gehäuseteils 4 eingreifen. Besonders bevorzugt ist dabei der Rastvorsprung 32 als umlaufender Vorsprung am oberen Rand 34 des Gehäuseteils 4 an dessen Außenwandung angeordnet, kann jedoch besonders bevorzugt nur als teilsweise ringförmig ausgebildeter Rastvorsprung 32 ausgebildet sein, so dass dort beispielsweise zwei, drei, vier oder mehr solcher teilweise kreisförmig umlaufend an der Außenwandung des oberen Randes 34 des Gehäuseteils 4 angeordnete Rastvorsprünge 32 vorgesehen sein können, die in die Ringnut 24 eingreifen. Durch die Verrastung ist vorzugsweise ein form- und/oder kraftschlüssiger Verbund zwischen dem Deckelteil 2 und dem Gehäuseteil 4 erzielt. In einer alternativen Ausgestaltung ist vorgesehen, dass das Deckelteil 2 durch festen Sitz des Verschlussteils im Gehäuseteil 4 auf dem Gehäuseteil 4 klemmend oder klebend mit diesem verbunden ist. Proximal des Verschlussteils 3 sind Einströmöffnungen 6 in der Seitenwandung des Gehäuseteils 4 vorgesehen, die ein Einströmen der Luft erlauben. Am proximalen Ende des Gehäuseteils 4 sind Ausströmöffnungen 9 vorgesehen, die beim Einatmen ein Ausströmen der eingeatmeten Luft in die Trachealkanüle beziehungsweise das Tracheostoma ermöglicht. Ein Ventilsitz 5 ist durch die Seitenwandung des Gehäuseteils 4 als umlaufender Vorsprung in Form einer Querschnittsverengung ausgebildet.

Der Filter 8, der passgenau in dem Gehäuseteil 4 angeordnet ist, befeuchtet die einströmende Luft, bevor diese die Trachealkanüle beziehungsweise das Tracheostoma erreicht. Proximal im Gehäuseteil 4 angeordnet ist ein Filtersitz 10, der ein Rutschen des Filters in die Trachealkanüle beziehungsweise Trachea verhindert. In einer alternativen Ausführungsform kann beispielsweise ein Filtersitz 10 nicht vorgesehen sein, sondern ausschließlich zur Verhinderung eines Durchrutschens des Filters 8 durch die mindestens eine Ausströmöffnung 9 ein Stützkreuz 11 vorgesehen sein, welches drei, vier, fünf oder mehr Stützarme aufweist. Der Filter 8 kann dann unmittelbar mit einer dieser zugewandten Oberfläche des Stützkreuzes 11 in Kontakt stehen beziehungsweise aufliegen. Die hier gezeigte Ausführungsform sieht ein Stützkreuz 11 vor ist, welches das Eindringen von Fremdkörpern, insbesondere des Filters 8, in die Trachea ebenfalls verhindert.

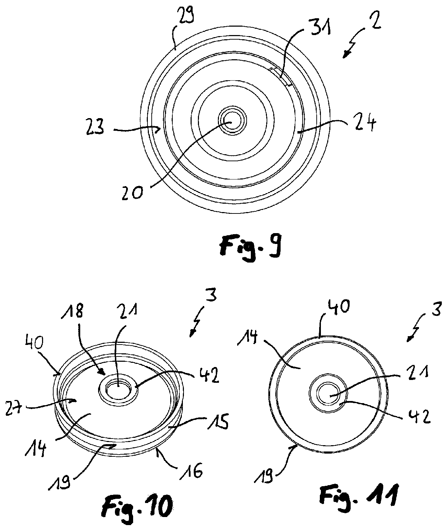

Das Deckelteil 2 weist auf seiner Innenseite 22 weiterhin einen Befestigungsvorsprung 20 in Form eines Befestigungsnoppens auf, der in eine Ausnehmung 21, die als Bohrung ausgestaltete Öffnung ausgeführt ist, des Verschlussteiles 3 eingreift und hierdurch das Verschlussteil 3 innenseitig des Deckelteils 2 oberhalb des Filters 8 im Gehäuseteil 4 hält. Das Verschlussteil 3 weist einen Außenrand 15 mit einer unteren Stirnfläche 16 und einer oberen Stirnfläche 40 (siehe Fig. 10) auf, wobei an der unteren Stirnfläche 16 ein Lippenelement 17 angeordnet ist, wobei das Lippenelement 17 in Verlängerung einer Außenwandung 19 des Außenrandes 15 auf der unteren Stirnfläche 16 angeordnet ist. Das Verschlussteil 3 ist kreis- oder scheibenförmig ausgebildet, wie insbesondere auch den Fig. 10 und 11 entnehmbar ist. Ein insbesondere durch die Ausnehmung 21 definierter Befestigungsbereich 18 des Verschlussteiles 3 ist weiterhin vorgesehen, der insbesondere umlaufend der Ausnehmung 21 einen Materialwulst 42 (siehe Fig. 10) aufweisen kann, insbesondere eine wulstförmige Materialverstärkung, um einen sicheren Halt des Verschlussteiles 3 am Befestigungsvorsprung 20 des Deckelteils 2 sicherzustellen.

Ausgehend von der kreisrund umlaufenden unteren Stirnfläche 16 des Verschlussteiles 3 zum Befestigungsbereich 18 hin weist das Verschlussteil 3 einen Mittelteil 14 auf mit einer unteren ansteigenden Oberfläche 26 und einer oberen Oberfläche 27, wobei letztere den Innenseiten 22 des Deckelteils 2 zugewandt ist. Die untere ansteigende Oberfläche 26 ist dem Gehäuseinneren und insbesondere dem Filter 8 zugewandt. Das Mittelteil 14 ist ansteigend von der unteren Stirnfläche 16 des Außenrandes 15 zum Befestigungsbereich 18 hin ausgebildet, das heißt insbesondere winklig zu einer Mittelachse 30 des Sprechventils 1 ausgebildet. Hierdurch wird zwischen Verschlussteil 3, insbesondere dessen unterer ansteigenden Oberfläche 26 des Mittelteils 14 und einer Oberseite des Filters 8 ein Raum 13 gebildet, in welchem Luft, die durch die Einströmöffnungen 6 eingeatmet wird, verwirbeln kann, so dass diese dann gleichmäßig durch den Filter 8 durchdringt bei Einatmung, das heißt auch eine gleichmäßige Befeuchtung und Erwärmung erfährt. Des Weiteren ist der Raum aufgrund der spezifischen Ausgestaltung des Verschlussteils 3 derart ausgestaltet, dass durch die Einströmöffnungen 6 einströmende Luft ohne Pfeifgeräusche eingeatmet wird. Die Einströmöffnungen 6 sind besonderes bevorzugt auf einer Ebene des Gehäuseteils 4 in dessen Inneren umlaufend angeordnet. Sie sind von Stegen, die das untere Gehäuseteil mit dem oberen Rand 34 des Gehäuseteils 4 verbinden, unterbrochen. Hierdurch wird eine hinreichende Stabilität dem Gehäuseteil 4 mitgegeben, welches im Übrigen auch den Fig. 5 bis 7 entnehmbar ist.

Die untere Stirnfläche 16 des Außenrandes 15 des Verschlussteils 3 als auch das Lippenelement 1 7 wirkt mit dem Ventilsitz 5 bei Überführung des Sprechventils 1 aus der Offenstellung in eine Schließstellung zusammen. Bei Aufbringung einer Kraft F in Richtung des in Fig. 1 gezeigten Pfeiles über eine Oberseite 28 des Deckelteils 2 verformt sich das aus einem elastischen Material gebildete Deckelteil 2 und ermöglicht so ein Zusammenwirken der unteren Stirnfläche 16 des Verschlussteils 3 beziehungsweise des Lippenelementes 17 mit der Ventilsitzfläche des Ventilsitzes 5. Aufgrund des Lippenelementes 17 erfolgt dabei eine weitgehend luftdichte Abdichtung des Ventiles in einer solchen Geschlossenstellung, so dass keine Luft durch die Einströmöffnungen 6 ein- oder ausgeatmet werden kann. Soweit kein Lippenelement 17 vorgesehen ist, sind bevorzugt die Stirnfläche 16 und die Ventilsitzfläche des Ventilsitzes 5 koplanar ausgebildet, so dass eine vollständige Dichtung zur Verfügung gestellt ist. Besonders bevorzugt ist das Verschlussteil 3 aus einem medizinischen Silikon gefertigt und weist eine gewisse Festigkeit, jedoch auch eine gewisse Elastizität noch auf, so dass aufgrund dieser Elastizität insbesondere des oder der Lippenelemente 17 die Dichtwirkung bei Überführung des erfindungsgemäßen Sprechventils 1 aus der Offenstellung in die Schließstellung unterstützt ist. Das Gehäuseteil 4 ist demgegenüber aus einem harten, rigidem Kunststoffmaterial hergestellt, welches nur eine geringe Elastizität aufweist, wenn überhaupt.

Mit dem Bezugszeichen 60 ist ein Bereich des Verschlussteils 3 bezeichnet, welcher oberhalb der oberen Oberfläche 27 des Mittelteils 14 des Verschlussteils 3 liegt. Damit soll verdeutlicht werden, dass dieser Bereich 60 auch verfüllt ausgebildet werden kann, das heißt bis zu diesem Niveau, welches durch die obere Stirnfläche des Materialwulstes 42 des Außenrandes 15 bestimmt wird, das Verschlussteil 3 verfüllt ausgebildet sein kann, das heißt in diesem Falle dann eine kreisförmige Scheibe mit einer Ausnehmung 21 in der Mitte darstellt, zu welcher hin sich dann eine untere ansteigende Oberfläche 26 des Mittelteils 14 erstreckt. Das Verschlussteil 3 kann als konusartig angesprochen werden.

Das Deckelteil 2 weist eine untere Stirnfläche 29 in dem Bereich auf, der vertikal einer Außenkontur des Gehäuseteils 4 überragt. Darüber hinaus überragt das Deckelteil 2 auch in horizontaler Richtung, das heißt senkrecht zu der Mittelachse 30, eine Außenkontur des Gehäuseteils 4. Hierdurch kann Luft, wie durch den Pfeil 12 kenntlich gemacht, durch die Einströmöffnungen 6 eingeatmet werden, wobei die eingeatmete Luft vorgewärmt wird aufgrund der Nähe der unteren Stirnfläche 29 zu einer Hautoberfläche des Trägers des Sprechventiles 1. Fig. 1 zeigt dabei nur eine mögliche Ausgestaltung des Deckelteils 2. Insbesondere kann auch vorgesehen sein, dass die untere Stirnfläche 29 des Deckelteils 2 in etwa auf Höhe des Ventilsitzes 5, der einer Unterkante der Einströmöffnung 6 vorzugsweise entspricht, endet. Auch hierdurch wird eine hinreichende Vorwärmung der Atemluft erzielt. Damit kann insbesondere auch das Sprechventil 1 besonders kleinbauend ausgestaltet werden, was einen besonderen Vorteil darstellt. Denn auch die Einströmöffnungen 6 können aufgrund der spezifischen Ausgestaltung des Verschlussteiles 3, welches Pfeifgeräusche vermeidet, mit einer geringen Höhe ausgestattet werden, bevorzugt mit einer Höhe, die gleich oder etwas kleiner als eine Höhe der Außenwandung 19 der Außenrandes 15 des Verschlussteils 5 ist. Hierdurch wird eine stets sichere Führung des Verschlussteiles 3 im Inneren des Gehäuseteils 4 sichergestellt, gleich ob in Offenstellung oder in Schließstellung.

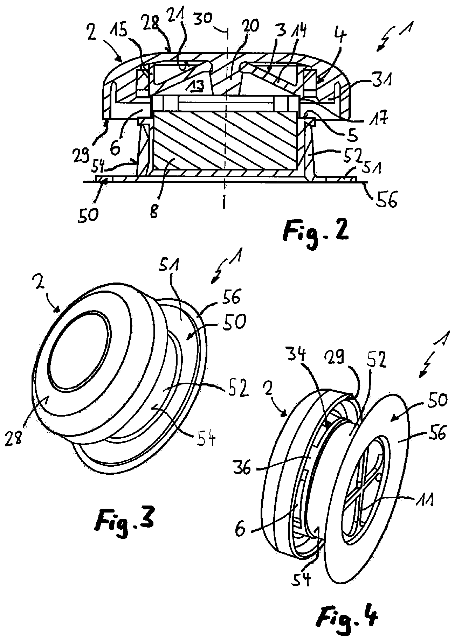

Fig. 2 zeigt eine alternative Ausführungsform eines erfindungsgemäßen Sprechventils 1 in einer Schnittansicht. Bei dieser schließt anders als bei der Ausführungsform gemäß Fig. 1 eine Oberseite des Filters 8 nicht mit einer Ventilsitzfläche des Ventilsitzes 5 ab, sondern überragt diese distal. Dies führt dazu, dass bei einer Überführung des Sprechventils 1 gemäß der Fig. 2 aus einer Offen- in eine Schließstellung das Verschlussteil 3 mit der unteren Stirnseite 16 der Außenwandung 15 mit dem Ventilsitz 5 zumindest teilweise zusammenwirkt, insbesondere im Bereich des Lippenelementes 17, wobei der Filter 8 geringfügig teilkomprimiert wird. Hierdurch wird ein zusätzlicher elastischer Effekt bei Rückführung des Schließventils aus der Schließstellung in die Offenstellung erzielt. Der Filter 8 ist, wie üblich, aus einem offenporigen Schaumstoffmaterial gebildet, welches eine gewisse Elastizität und damit ein gewisses Rückstellvermögen aufweist, und daher das Deckelteil 2 in seinem Rückstellvermögen aufgrund der Verwendung eines elastischen Materiales unterstützt.

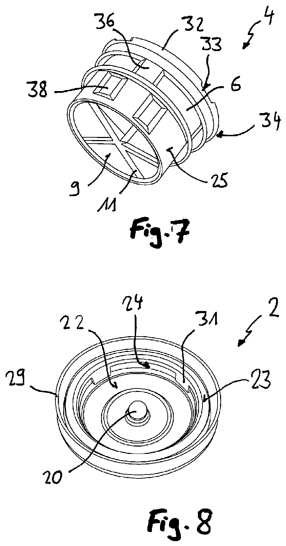

Ansonsten ist bei der Ausführungsform gemäß Fig. 2 im Unterschied zu derjenigen gemäß Fig. 1 eine untere Stirnfläche 29 des Deckelteils 2 endend etwa auf der Höhe der Ventilsitzfläche des Ventilsitzes 5, wie bereits in Zusammenhang mit der Ausführungsform gemäß Fig. 1 in einer ersten Alternative beschrieben. Das Rastelement 23 des Deckelteils 2, angeordnet auf dessen Innenseite 22, ist anders ausgebildet. Der Schnitt gemäß Fig. 2 zeigt dabei einen Schnitt durch Verfüllelemente 31 desselben, die insbesondere Fig. 8 gut entnehmbar sind. Damit weist die umlaufende Nut 24 des Deckelteils 2, gebildet durch das Rastelement 23 und eine Teilfläche der Innenseite 22 des Deckelteils 2, keine vollständig umlaufende, sondern nur teilweise umlaufende Ausgestaltung, unterbrochen durch insgesamt vier Verfüllelemente 31, auf. Es wird ein passgenaues Anordnen des Deckelteils 2 auf dem Gehäuseteil 4 notwendig.

Wie insbesondere den Fig. 5 bis 7 entnommen werden kann, weist das Gehäuseteil 4 hierfür vier Rastvorsprünge 32.1 bis 32.4 auf, die in die entsprechenden Teilbereiche der nur teilweise ringförmig umlaufend ausgebildeten Ringnut 24 eingreifen.

Der Befestigungsvorsprung 20 ist noppenartig mit einer umlaufenden Ringnut ausgebildet, in welche der Befestigungsbereich 18 des Verschlussteils 3 eingreifbar ist. Durch die etwas verdickte Ausbildung des Befestigungsvorsprungs 20, in Verbindung mit der Elastizität des Materiales des Deckelteils 2, wird hierdurch eine sichere Anordnung des Verschlussteiles 3 an der Innenseite 22 des Deckelteils 2 ermöglicht.

Wie auch bei der Ausführungsform gemäß der Fig. 1 ist das Deckelteil 24 aus einem elastischen Material gebildet, wohingegen das Verschlussteil 3 aus einem erheblich rigiderem, das heißt festerem Material gebildet ist als das Deckelteil 2. Gleichwohl weist das Verschlussteil 3 insbesondere eine gewisse Elastizität noch auf, um eine möglichst gute Dichtwirkung bei Überführung des Sprechventiles 1 in die Schließstellung bei Zusammenwirkung mit der Ventilsitzfläche des Ventilsitzes 5 zu erzielen.

Bei der Ausführungsform der Fig. 2 ist auch ein Tracheostomapflaster 50 gezeigt, in welches das Gehäuseteiles 4 des Sprechventiles 1 in eine Aufnahme 52 mit einer Außenwandung 54 aufnehmbar ist. Die Aufnahme weist eine Bodenplatte 51 auf. Das Tracheostomapflaster 50 weist proximal ein insbesondere ringförmig ausgebildetes Klebeband 56, angeordnet an eine Unterseite der Bodenplatte 51, auf, mittels welchem dieses auf der Haut eines Trägers befestigt werden kann. Alternativ kann aber beispielsweise das Sprechventil gemäß den Fig. 1 und 2 auch aufgenommen sein in einer Trachealkanüle, die mit ihrer Aufnahme die Außenwandung 25 des Gehäuseteils 4 umfasst.

Die Fig. 3 und 4 zeigen perspektivische Ausgestaltungen der zweiten Ausführungsform gemäß Fig. 2. Insbesondere aus Fig. 4 wird die Ausgestaltung des Stützkreuzes 11 ersichtlich, welches den Filter 8 trägt. Im Unterschied zu der Ausführungsform gemäß Fig. 1 weist die Ausführungsform gemäß den Fig. 2 bis 4 keinen Filtersitz 10 auf, der Filter 8 wird durch das Stützkreuz 11 gehalten und an einem Durchrutschen beim Atmen in die Trachea gehindert.

Die Fig. 5 und 7 zeigen das Gehäuseteil 4, wie dieses beispielsweise in der zweiten Ausführungsform gemäß den Fig. 2 bis 4 des erfindungsgemäßen Sprechventils 1 einsetzbar ist. Dieses weist insgesamt 4 Einströmöffnungen 6 auf, die unterbrochen sind durch insgesamt vier Stege 36, die einen unteren Teil des Gehäuseteils 4 mit dem oberen Rand 34 desselben verbinden. Am oberen Rand 34 sind insgesamt vier Rastvorsprünge 32.1 bis 32.4 angeordnet, welche mit der zumindest teilweise umlaufenden Ringnut 24 eines Deckelteils 2 zusammenwirken können wir bereits vorstehend beschrieben. Auch ist die Ausgestaltung des Stützkreuzes 11 mit den insgesamt vier Stützarmen und dementsprechend gebildeten vier Austrittsöffnungen 9 gut ersichtlich. Der Ventilsitz 5 bildet eine Ventilsitzfläche aufgrund einer Querschnittsverengung des Gehäuseteils 4. Zwischen den vier Rastvorsprüngen 32.1 bis 32.4 sind Ausnehmungen 33 angeordnet. Es kann jedoch auch vorgesehen sein, dass diese beispielsweise weggelassen werden, das heißt insgesamt nur ein umlaufender Rastvorsprung 32 ausgebildet ist. Fig. 7 sind noch Rasthilfen 38 zu entnehmen, die der Aufnahme des erfindungsgemäßen Sprechventils in ein Tracheostomapflaster 50, wie in den Fig. 2 bis 4 gezeigt, oder eine Trachealkanüle dienen beziehungsweise eine solche erleichtern. Dieses sind aufgenommen in der Gehäusewandung 25 des Gehäuseteils 4.

Die Fig. 8 und 9 zeigen ein Deckelteil 2, wie dieses in dem erfindungsgemäßen Sprechventil 1, insbesondere bei demjenigen gemäß der zweiten Ausführungsform nach den Fig. 2 bis 4, eingesetzt werden kann. Das dort gezeigte Deckelteil 2 ist dabei so ausgebildet, dass es eine Ringnut 24 aufweist, welche nur teilweise umlaufend ringförmig ausgebildet ist, da diese von Verfülletementen 31, und zwar insgesamt vier, unterbrochen ist, so dass das dort gezeigte Deckelteil 2 passgenau auf einem Gehäuseteil 4 gemäß den Fig. 5 bis 7 angeordnet werden muss. Die Verfüllelemente 31 können jedoch auch weggelassen werden, so dass ein solches passgenaues Anordnen nicht mehr notwendig ist. Ansonsten ist die Funktion und die Ausgestaltung des Deckelteils gemäß den Fig. 8 und 9 bereits in Zusammenhang mit der ersten und zweiten Ausführungsform des erfindungsgemäßen Sprechventils 1 gemäß den Fig. 1 bis 4 beschrieben worden.

Die Fig. 10 und 11 zeigen nun ein erfindungsgemäßes Verschlussteil 3, wie dieses insbesondere in einem Sprechventil 1 gemäß den Fig. 1 bis 4 eingesetzt werden kann. Deutlich ist ein Befestigungsbereich 18 zu erkennen mit einer Ausnehmung 21, umgeben von einem Materialwulst 42. Darüber hinaus weist das Verschlussteil 3 ein Außenrand 15 mit einer unteren Stirnfläche 16 und einer oberen Stirnfläche 40 auf als auch einen von der unteren Stirnfläche 16 zum Befestigungsbereich 18 hin ansteigenden Mittelteil 14. Der Mittelteil 14 bildet dabei eine obere Oberfläche 27 und eine untere Oberfläche 26 auf, wobei oberhalb der oberen Oberfläche 27 bis zu der oberen Stirnfläche 40 des Außenrandes 15 und zum Materialwulst 42 hin der solcher Maßen definierte Bereich auch verfüllt ausgebildet sein kann, wie durch das Bezugszeichen 60 in Fig. 1 prinzipienhaft verdeutlicht. Der Außenrand 15 weist eine Außenwandung 19 auf, welche insbesondere einer sicheren Führung des Verschlussteiles innerhalb eines Sprechventiles gemäß einem der Ausführungsbeispiele 1 bis 4 dient bei Überführung aus einer Offenstellung in eine Schließstellung und aus der Schließstellung wieder zurück in die Offenstellung.

Durch die genannten Maßnahmen, aber auch einzeln durch jede für sich, wird auf einfache Weise eine sichere Abdichtung des Sprechventils 1 erreicht. Eine Rückstellkraft ist durch Auswahl des Materials, der Form und des Deckelteils bedingt und kann eingestellt werden. Wird die Kraft auf das Deckelteil gelöst beziehungsweise lässt der Benutzer von dem Deckelteil ab, führt diese Rückstellkraft zum Wiederöffnen des Ventils.

## Patentansprüche

1. Sprechventil (1) für Laryngektomierte oder Tracheotomierte mit einem Deckelteil (2), einem Gehäuseteil (4) und einem im Gehäuseteil (4) angeordneten Filter (8), wobei das Deckelteil (2) mittels einer Rastverbindung an dem Gehäuseteil (4) angeordnet ist, wobei das Gehäuseteil (4) distal einen Ventilsitz (5) umfasst, wobei das Deckelteil (2) ein auf einer Innenseite (22) desselben angeordnetes, dem Filter (8) zugewandtes Verschlussteil (3) umfasst, wobei das Deckelteil (2) ein elastisches Material umfasst, wobei mittels Verformens zumindest eines Bereiches des Deckelteils (2) das Verschlussteil (3) das Sprechventil (1) distal des Filters (8) verschließt, wobei das Verschlussteil (3) auf einer unteren Stirnfläche (16) eines Außenrandes (15) mindestens ein Lippenelement (17) aufweist, wobei das Lippenelement (17) bei Betätigung des Sprechventils (1) mit dem Ventilsitz (5) zusammenwirkt, wobei das Verschlussteil (3) kreisförmig ausgebildet ist und ausgehend von der unteren Stirnfläche (16) des Außenrandes (15) zu einem mittig angeordneten Befestigungsbereich (18) eine ansteigende Oberfläche (26) eines Mittelteils (14) aufweist, und das Gehäuseteil (4) distal des Ventilsitzes (5) Einströmöffnungen (6) umfasst, die bei Betätigung des Sprechventils (1) mittels des Verschlussteils (3) verschließbar sind, und zumindest ein elastischer Bereich des Deckelteils (2) als Rückstellelement fungiert.

2. Sprechventil gemäß Anspruch 1, **dadurch gekennzeichnet, dass** der Außenrand (15) eine Außenwandung (19) aufweist, die im Wesentlichen parallel zu einer Innenkontur des Gehäuseteils (4) angeordnet ist.

3. Sprechventil gemäß einem oder mehreren der vorhergehenden Ansprüche, dadurch gegenzeichnet, dass der Befestigungsbereich (18) eine Ausnehmung (21) umfasst, in welche ein Befestigungsvorsprung (20) des Deckelteils (2) eingreifbar ist.

4. Sprechventil gemäß einem oder mehreren der vorhergehenden Ansprüche, **dadurch gekennzeichnet, dass** die untere Stirnfläche (16) des Verschlussteils (3) zumindest teilweise mit dem Ventilsitz (5) zusammenwirkt.

5. Sprechventil gemäß einem oder mehreren der vorhergehenden Ansprüche, **dadurch gekennzeichnet, dass** die mindestens eine Einströmöffnung (6) in Richtung einer Mittelachse (30) des Ventils (1) eine Höhe aufweist, die etwa gleich oder kleiner als eine Höhe des Außenrandes (15) des Verschlussteils (3) ist.

6. Sprechventil gemäß einem oder mehreren der vorhergehenden Ansprüche, **dadurch gekennzeichnet, dass** das Deckelteil (2) auf dem Gehäuseteil (4) lösbar angeordnet ist.

7. Sprechventil gemäß einem oder mehreren der vorhergehenden Ansprüche, **dadurch gekennzeichnet, dass** das Deckelteil (2) auf seiner Innenseite (22) mindestens ein Rastelement (23) aufweist, welches mindestens eine zumindest teilweise umlaufende Nut (24) bildet.

8. Sprechventil gemäß einem oder mehreren der vorhergehenden Ansprüche, **dadurch gekennzeichnet, dass** das Verschlussteil (3) aus einem rigideren Material als das Deckelteil (2) gebildet ist.

9. Sprechventil gemäß einem oder mehreren der vorhergehenden Ansprüche, **dadurch gekennzeichnet, dass** das Deckelteil (2) das Gehäuseteil (4) zumindest teilweise außenseitig überragt.

10. Sprechventil gemäß einem oder mehreren der vorhergehenden Ansprüche, **dadurch gekennzeichnet, dass** das Deckelteil (2) das Gehäuseteil (4) lateral und vertikal überragt.

11. Sprechventil gemäß einem oder mehreren der vorhergehenden Ansprüche, **dadurch gekennzeichnet, dass** der Filter (8) mit Übergangspassung oder Übermaßpassung in dem Gehäuseteil (4) angeordnet ist.

12. Sprechventil gemäß einem oder mehreren der vorhergehenden Ansprüche, **dadurch gekennzeichnet, dass** der Filter (8) durch die Betätigung des Sprechventils (1) teilweise komprimiert wird.

13. Verschlussteil (3) für ein Sprechventil (1) für Laryngektomierte oder Tracheotomierte gemäß einem der Ansprüche 1-12, **dadurch gekennzeichnet, dass** das Verschlussteil (3) auf einer unteren Stirnfläche (16) eines Außenrandes (15) mindestens ein Lippenelement (17) aufweist, und wobei das Verschlussteil (3) kreisförmig ausgebildet ist, und ausgehend von der unteren Stirnfläche (16) des Außenrandes (15) zu einem mittig angeordneten Befestigungsbereich (18) eine ansteigende Oberfläche (26) eines Mittelteils (14) aufweist.

## Claims

1. Speaking valve (1) for laryngectomized or tracheostomized patients, having a cover part (2), a housing part (4), and a filter (8) disposed in the housing part (4), wherein the cover part (2) is disposed at the housing part (4) by means of a latching connection, wherein the housing part (4) comprises a valve seat (5) at its distal end, wherein the cover part (2) comprises a closure part (3) disposed on an inner surface (22) thereof, facing toward the filter (8), wherein the cover part (2) comprises an elastic material, wherein by means of deformation of at least one region of the cover part (2), the closure part (3) closes the speaking valve (1) at the end distal to the filter (8), wherein the closure part (3) has at least one lip element (17) on a lower surface (16) of an outer edge (15), wherein the lip element (17) interacts with the valve seat (5) upon actuating the speaking valve (1), wherein the closure part (3) has a circular design, and has a rising surface (26) of a middle part (14), starting from the lower surface (16) of the outer edge (15) to an attachment region (18) disposed centrally, and the housing part (4) comprises inflow openings (6) at the end distal to the valve seat (5), which can be locked by means of the closure part (3) upon actuating the speaking valve (1), and at least one elastic region of the cover part (2) acts as a resetting element.

2. Speaking valve according to claim 1, **characterized in that** the outer edge (15) has an outer wall (19) which is disposed substantially parallel to an inner contour of the housing part (4).

3. Speaking valve according to one or more of the preceding claims, **characterized in that** the attachment region (18) comprises a cavity (21), in which an attachment projection (20) of the cover part (2) can engage.

4. Speaking valve according to one or more of the preceding claims, **characterized in that** the lower surface (16) of the closure part (3) interacts at least in part with the valve seat (5).

5. Speaking valve according to one or more of the preceding claims, **characterized in that** the at least one inflow opening (6) has a height in the direction of the central axis (30) of the valve (1), which is basically equal to or less than a height of the outer edge (15) of the closure part (3).

6. Speaking valve according to one or more of the preceding claims, **characterized in that** the cover part (2) is releasably disposed on the housing part (4).

7. Speaking valve according to one or more of the preceding claims, **characterized in that** the cover part (2) has at least one latching element (23) on its inner surface (22), which forms at least one at least partially circumferential groove (24).

8. Speaking valve according to one or more of the preceding claims, **characterized in that** the closure part (3) is made of a material that is more rigid than the cover part (2).

9. Speaking valve according to one or more of the preceding claims, **characterized in that** the cover part (2) extends at least in part over the outside of the housing part (4).

10. Speaking valve according to one or more of the preceding claims, **characterized in that** the cover part (2) extends over the housing part (4) laterally and vertically.

11. Speaking valve according to one or more of the preceding claims, **characterized in that** the filter (8) is disposed in the housing part (4) with a transition fit or interference fit.

12. Speaking valve according to one or more of the preceding claims, **characterized in that** the filter (8) is partially compressed by the actuation of the speaking valve (1).

13. Closure part (3) for a speaking valve (1) for laryngectomized or tracheostomized patients according to anyone of the claims 1 - 12, **characterized in that** the closure part (3) has at least one lip element (17) on a lower surface (16) of an outer edge (15), and wherein the closure part (3) has a circular design, and has a rising surface (26) of a middle part (14), starting from the lower surface (16) of the outer edge (15) to a centrally disposed attachment region (18).

## Revendications

1. Valve de phonation (1) pour laryngectomisés ou trachéotomisés qui comporte une partie couvercle (2), une partie boitier (4) et un filtre (8) placé dans la partie boitier (4), ladite partie couvercle (2) étant disposée au moyen d'une liaison d'encliquetage au niveau de ladite partie boitier (4), ladite partie boitier (4) comportant distalement un siège de valve (5), ladite partie couvercle (2) comportant une partie verrouillage (3) orientée vers ledit filtre (8), disposée sur un côté interne (22) de celle-ci, ladite partie couvercle (2) étant constituée d'une matière élastique, ladite partie verrouillage (3) fermant ladite valve de phonation (1) à distance dudit filtre (8) par déformation d'au moins une zone de ladite partie couvercle (2), ladite partie verrouillage (3) comportant sur une face frontale (16) inférieure d'un bord externe (15) au moins un élément de lèvre (17), ledit élément de lèvre (17) interagissant avec ledit siège de valve (5) lors de l'actionnement de ladite valve de phonation (1), ladite partie verrouillage (3) étant circulaire, et comportant en partant de ladite face frontale (16) inférieure dudit bord externe (15), vers une zone de fixation (18) disposée au milieu, une surface (26) croissante d'une partie centrale (14), et ladite partie boitier (4) comprenant à distance dudit siège de valve (5) des ouvertures d'admission (6) qui, lors de l'actionnement de ladite valve de phonation (1), peuvent être fermées au moyen de ladite partie verrouillage (3) et au moins une zone élastique de ladite partie couvercle (2) fonctionnant en tant qu'élément de rappel.

2. Valve de phonation selon la revendication 1, **caractérisée en ce que** ledit bord externe (15) comporte une paroi extérieure (19) qui est disposée essentiellement parallèlement à un contour intérieur de ladite partie boitier (4).

3. Valve de phonation selon l'une ou plusieurs des précédentes revendications, **caractérisée en ce que** ladite zone de fixation (18) comporte un évidement (21), dans lequel une saillie de fixation (20) de ladite partie couvercle (2) peut être en prise.

4. Valve de phonation selon l'une ou plusieurs des précédentes revendications, **caractérisée en ce que** ladite face frontale (16) inférieure de ladite partie verrouillage (3) interagit au moins en partie avec ledit siège de valve (5).

5. Valve de phonation selon l'une ou plusieurs des précédentes revendications, **caractérisée en ce qu'**au moins une ouverture d'admission (6) comporte en direction d'un axe médian (30) de ladite valve (1) une hauteur qui est à peu près inférieure ou égale à une hauteur dudit bord externe (15) de ladite partie verrouillage (3).

6. Valve de phonation selon l'une ou plusieurs des précédentes revendications, **caractérisée en ce que** ladite partie couvercle (2) est disposée, de manière amovible, sur ladite partie boitier (4).

7. Valve de phonation selon l'une ou plusieurs des précédentes revendications, **caractérisée en ce que** ladite partie couvercle (2) comporte sur son coté interne (22) au moins un élément d'encliquetage (23), qui forme au moins une rainure (24) au moins en partie circulaire.

8. Valve de phonation selon l'une ou plusieurs des précédentes revendications, **caractérisée en ce que** ladite partie verrouillage (3) est formée, en tant que ladite partie couvercle (2), en un matériau rigide.

9. Valve de phonation selon l'une ou plusieurs des précédentes revendications, **caractérisée en ce que** ladite partie couvercle (2) dépasse ladite partie boitier (4) au moins en partie sur un côté extérieur.

10. Valve de phonation selon l'une ou plusieurs des précédentes revendications, **caractérisée en ce que** ladite partie couvercle (2) dépasse ladite partie boitier (4) latéralement et verticalement.

11. Valve de phonation selon l'une ou plusieurs des précédentes revendications, **caractérisée en ce que** ledit filtre (8) est disposé avec un ajustement de transition ou ajustement avec serrage dans ladite partie boitier (4).

12. Valve de phonation selon l'une ou plusieurs des précédentes revendications, **caractérisée en ce que** ledit filtre (8) est comprimé en partie par l'actionnement de ladite valve de phonation (1).

13. Partie verrouillage (3) pour une valve de phonation (1) pour laryngectomisés ou trachéotomisés, selon l'une des revendications 1 à 12, **caractérisée en ce que** ladite partie verrouillage (3) comporte sur une face frontale (16) inférieure d'un bord externe (15) au moins un élément de lèvre (17), ladite partie verrouillage (3) étant circulaire, et comportant en partant de la face frontale (16) inférieure dudit bord externe (15), vers une zone de fixation (18) disposée au milieu, une surface (26) croissante d'une partie centrale (14).
